# EUROPÄISCHE PATENTANMELDUNG

(11) **EP 4 834 387 A1**
(43) Veröffentlichungstag der Anmeldung: **04.11.2026**
(21) Anmeldenummer: 26174182.1
(22) Anmeldetag: 23.04.2026
(51) Int. Cl.: A61M 1/16, A61M 1/34

(54) **VERFAHREN ZUR ZUSTANDSÜBERWACHUNG EINER VORRICHTUNG ZUR EXTRAKORPORALEN BLUTBEHANDLUNG UND VORRICHTUNG ZUR EXTRAKORPORALEN BLUTBEHANDLUNG**

(30) Priorität: 30.04.2025 DE 102025116818
(71) Anmelder: B. Braun Avitum AG, 34212 Melsungen (DE)
(72) Erfinder: Riemenschneider, Heiko, 34327 Wagenfurth (DE)
(74) Vertreter: Patentanwälte Ruff, Wilhelm, Beier, Dauster & Partner mbB

(57) **Zusammenfassung**

Verfahren zur Zustandsüberwachung einer Vorrichtung (100) zur extrakorporalen Blutbehandlung, wobei die Vorrichtung (100) zur extrakorporalen Blutbehandlung aufweist:
- eine elektrische Dialysierflüssigkeitspumpe (1) zum Fördern von Dialysierflüssigkeit (2),
- einen ersten Dialysierflüssigkeitsfilter (3) mit einer Filtermembran (9), der in einer Spülbetriebsart und in einer Filterbetriebsart betreibbar ist, und
- eine Magnetventilanordnung (4, 5, 6, 7, 8) zur Steuerung eines Flusspfads der geförderten Dialysierflüssigkeit (2),
wobei das Verfahren die Schritte aufweist:
- Einstellen der Magnetventilanordnung (4, 5, 6, 7, 8) derart, dass der erste Dialysierflüssigkeitsfilter (3) seine Spülbetriebsart einnimmt,
- Fördern von Dialysierflüssigkeit (2) durch den ersten Dialysierflüssigkeitsfilter (3) in seiner Spülbetriebsart,
- Messen einer ersten Stromaufnahme der elektrischen Dialysierflüssigkeitspumpe (1),
- Vergleichen den gemessenen ersten Stromaufnahme mit einem ersten Schwellenwert und
- falls die gemessene erste Stromaufnahme größer als der erste Schwellenwert ist, Feststellen eines Defekts der elektrischen Dialysierflüssigkeitspumpe (1).

## Beschreibung

Der Erfindung liegt die Aufgabe zugrunde, ein Verfahren zur Zustandsüberwachung einer Vorrichtung zur extrakorporalen Blutbehandlung und eine Vorrichtung zur extrakorporalen Blutbehandlung zur Verfügung zu stellen, die eine möglichst zuverlässige und kostengünstige Überwachung ermöglichen.

Das erfindungsgemäße Verfahren dient zur Zustandsüberwachung einer Vorrichtung zur extrakorporalen Blutbehandlung, beispielsweise in Form einer Dialysemaschine. Die Vorrichtung zur extrakorporalen Blutbehandlung weist eine elektrische Dialysierflüssigkeitspumpe zum Fördern von Dialysierflüssigkeit auf. Die Vorrichtung weist weiter einen ersten Dialysierflüssigkeitsfilter mit einer Filtermembran auf, der in einer Spülbetriebsart und in einer Filterbetriebsart betreibbar ist. Die Vorrichtung weist weiter eine Magnetventilanordnung mit mehreren Magnetventilen zur Steuerung eines Flusspfads der geförderten Dialysierflüssigkeit auf. Selbstverständlich weist die Vorrichtung zur extrakorporalen Blutbehandlung weitere herkömmliche Komponenten auf, die jedoch aus Gründen der Fokussierung unerwähnt bleiben können.

Erfindungsgemäß wird die Magnetventilanordnung derart eingestellt, dass der erste Dialysierflüssigkeitsfilter seine Spülbetriebsart einnimmt. Es wird dann Dialysierflüssigkeit durch den ersten Dialysierflüssigkeitsfilter in seiner Spülbetriebsart gefördert, eine erste Stromaufnahme der elektrischen Dialysierflüssigkeitspumpe gemessen und die gemessene erste Stromaufnahme mit einem ersten Schwellenwert verglichen. Falls die gemessene erste Stromaufnahme größer als der erste Schwellenwert ist, wird ein Defekt der elektrischen Dialysierflüssigkeitspumpe festgestellt. Der erste Schwellenwert kann beispielsweise rechnerisch oder empirisch ermittelt werden. Der erste Schwellenwert wird derart gewählt, dass er bei einer ordnungsgemäß funktionierenden Dialysierflüssigkeitspumpe stets unterschritten wird. Aufgrund der Spülbetriebsart sind Einflüsse des Dialysierflüssigkeitsfilters auf die Stromaufnahme der Dialysierflüssigkeitspumpe zu vernachlässigen. Eine Stromerhöhung zeigt dann beispielweise an, dass ein Pumpenkopf mit höherer Reibung dreht und beginnt zu klemmen. Die Zustandsüberwachung überwacht folglich den Zustand der Dialysierflüssigkeitspumpe, d.h. überwacht, ob die Dialysierflüssigkeitspumpe ordnungsgemäß funktioniert oder nicht.

In einer Ausführungsform wird die Magnetventilanordnung derart eingestellt, dass der erste Dialysierflüssigkeitsfilter seine Filterbetriebsart einnimmt, Dialysierflüssigkeit wird durch den ersten Dialysierflüssigkeitsfilter in seiner Filterbetriebsart gefördert, eine zweite Stromaufnahme der elektrischen Dialysierflüssigkeitspumpe wird gemessen und ein Zustand bzw. ein Verschleiß der Filtermembran des ersten Dialysierflüssigkeitsfilters wird in Abhängigkeit von der gemessenen zweiten Stromaufnahme ermittelt. Der Zustand der Filtermembran kann beispielsweise ein Maß der Verschmutzung der Filtermembran abbilden, beispielswiese aufgrund von Verkalkung und/oder Ablagerung von gefilterten Stoffen. Beispielsweise kann eine Stromaufnahme bei einer unbenutzten Filtermembran gemessen und dann als ein unterer Referenzwert gespeichert werden. Weiter kann eine Stromaufnahme bei einer benutzten und nahezu zugesetzten Filtermembran gemessen werden und als ein oberer Referenzwert gespeichert werden. Der Zustand einer Filtermembran kann dann durch Interpolation zwischen dem unteren und dem oberen Referenzwert basierend auf der während des Betriebs gemessenen zweiten Stromaufnahme abgeschätzt werden.

In einer Ausführungsform wird die gemessene zweite Stromaufnahme mit einem zweiten Schwellenwert verglichen. Falls die gemessene zweite Stromaufnahme größer als der zweite Schwellenwert ist, wird ein Fördervolumenstrom der Dialysierflüssigkeit auf einen Maximalwert begrenzt und eine Empfehlung zum Wechseln der Filtermembran des ersten Dialysierflüssigkeitsfilters bzw. des gesamten ersten Dialysierflüssigkeitsfilters ausgegeben. Auf diese Weise kann verhindert werden, dass eine laufende Therapie unterbrochen werden muss. Beispielsweise kann der Fördervolumenstrom der Dialysierflüssigkeit bzw. der Dialysatfluss von beispielsweise 800 ml/min auf 700 ml/min abgesenkt werden. Ohne eine solche Maßnahme würde das Dialysesystem "kollabieren" und es käme zu einem Therapieabbruch.

In einer Ausführungsform weist die Vorrichtung zur extrakorporalen Blutbehandlung weiter einen zweiten Dialysierflüssigkeitsfilter mit einer Filtermembran auf, der in einer Spülbetriebsart und in einer Filterbetriebsart betreibbar ist. Das Verfahren weist die weiteren Schritte auf: Einstellen der Magnetventilanordnung derart, dass der erste Dialysierflüssigkeitsfilter und der zweite Dialysierflüssigkeitsfilter jeweils ihre Filterbetriebsart einnehmen und die Dialysierflüssigkeit durch den ersten und durch den zweiten Dialysierflüssigkeitsfilter gefördert wird, Messen einer dritten Stromaufnahme der elektrischen Dialysierflüssigkeitspumpe, Überprüfen, ob die gemessene dritte Stromaufnahme in etwa das Zweifache, d.h. beispielsweise das 1,2-fache bis 2,8-fache, der gemessenen zweiten Stromaufnahme beträgt, und falls die gemessene dritte Stromaufnahme nicht in etwa das Zweifache der gemessenen zweiten Stromaufnahme beträgt, Feststellen eines Defekts des zweiten Dialysierflüssigkeitsfilters. Andernfalls wird kein Defekt festgestellt.

In einer Ausführungsform weist die Vorrichtung zur extrakorporalen Blutbehandlung weiter einen Substitutions-Port auf, wobei das Verfahren die Schritte aufweist: Einstellen der Magnetventilanordnung derart, dass der erste Dialysierflüssigkeitsfilter und der zweite Dialysierflüssigkeitsfilter jeweils ihre Filterbetriebsart einnehmen und die Dialysierflüssigkeit durch den ersten Dialysierflüssigkeitsfilter, durch den zweiten Dialysierflüssigkeitsfilter und intern durch den Substitutions-Port gefördert wird, Messen einer vierten Stromaufnahme der elektrischen Dialysierflüssigkeitspumpe, Überprüfen, ob die gemessene vierte Stromaufnahme mit der der gemessenen dritten Stromaufnahme übereinstimmt, und falls die gemessene vierte Stromaufnahme nicht mit der der gemessenen dritten Stromaufnahme übereinstimmt, Feststellen eines Verschlusses bzw. Teil-Verschlusses des Substitutions-Ports. Andernfalls wird kein Defekt festgestellt.

In einer Ausführungsform werden der erste Schwellenwert und der zweite Schwellenwert bei bekannten Referenzbedingungen ermittelt.

Die erfindungsgemäße Vorrichtung zur extrakorporalen Blutbehandlung ist zur Durchführung eines oben beschriebenen Verfahrens ausgebildet.

Die Erfindung wird nachfolgend unter Bezugnahme auf die Zeichnungen detailliert beschrieben. Hierbei zeigt:
- Fig. 1: ein schematisches Blockschaltbild einer erfindungsgemäßen Vorrichtung zur extrakorporalen Blutbehandlung und
- Fig. 2: ein exemplarisches Flussdiagramm eines erfindungsgemäßen Verfahrens zur Zustandsüberwachung der in Fig. 1 gezeigten Vorrichtung zur extrakorporalen Blutbehandlung.

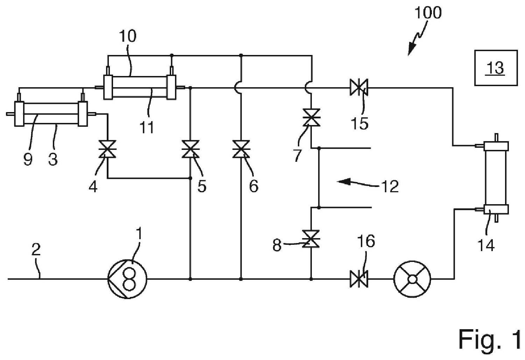

Fig. 1 zeigt ein schematisches Blockschaltbild einer erfindungsgemäßen Vorrichtung 100 zur extrakorporalen Blutbehandlung. Die erfindungsgemäßen Vorrichtung 100 zur extrakorporalen Blutbehandlung weist auf: eine elektrische Dialysierflüssigkeitspumpe 1 zum Fördern von Dialysierflüssigkeit 2, einen ersten Dialysierflüssigkeitsfilter 3 mit einer Filtermembran 9, der in einer Spülbetriebsart und in einer Filterbetriebsart betreibbar ist, einen zweiten Dialysierflüssigkeitsfilter 10 mit einer Filtermembran 11, der in einer Spülbetriebsart und in einer Filterbetriebsart betreibbar ist, eine Magnetventilanordnung mit Magnetventilen 4, 5, 6, 7, 8, 15 und 16 zur Steuerung eines Flusspfads der geförderten Dialysierflüssigkeit 2, einen Substitutions-Port 12, eine Steuereinheit 13 und einen Dialysator 14.

Der erste Dialysierflüssigkeitsfilter 3 kann beispielsweise ein Dialysefilter (DF-Filter) sein und der zweite Dialysierflüssigkeitsfilter 10 kann beispielsweise ein Hämodialyse-Filter (HDF-Filter) sein.

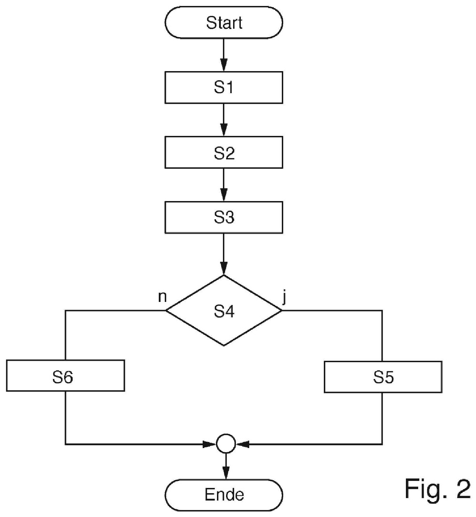

Die Zustandsüberwachung der erfindungsgemäßen Vorrichtung 100 zur extrakorporalen Blutbehandlung wird nachfolgend, auch unter Bezugnahme auf das in Fig. 2 gezeigte Flussdiagramm, beschrieben.

In einem Schritt S1 wird die Magnetventilanordnung 4, 5, 6, 7, 8, 15 und 16 derart eingestellt, dass der erste Dialysierflüssigkeitsfilter 3 seine Spülbetriebsart einnimmt. Hierzu wird das Magnetventil 4 geöffnet und die Magnetventile 5, 6, 7, 8, 15 und 16 werden geschlossen.

In einem Schritt S2 wird durch Aktivieren der Dialysierflüssigkeitspumpe 1 Dialysierflüssigkeit 2 durch den ersten Dialysierflüssigkeitsfilter 3 in seiner Spülbetriebsart, d.h. an der Filtermembran 9 vorbei, zur Dialysierflüssigkeitspumpe 1 geführt.

In einem Schritt S3 wird eine erste Stromaufnahme der elektrischen Dialysierflüssigkeitspumpe 1 gemessen.

In einem Schritt S4 wird die gemessene erste Stromaufnahme mit einem ersten Schwellenwert verglichen. Falls die gemessene erste Stromaufnahme größer als der erste Schwellenwert ist, dargestellt als "j", wird in einem Schritt S5 ein Defekt der elektrischen Dialysierflüssigkeitspumpe 1 festgestellt, da aufgrund des minimalen Flusswiderstands die Stromaufnahme der Dialysierflüssigkeitspumpe 1 minimal sein müsste. Falls die gemessene erste Stromaufnahme kleiner als oder gleich wie der erste Schwellenwert ist, dargestellt als "n", wird in einem Schritt S6 die Dialysierflüssigkeitspumpe 1 als fehlerfrei bewertet.

Neben dieser Zustandsüberwachung sind weitere Prüfschritte möglich, die nachfolgend beschrieben werden.

Die Magnetventilanordnung 4, 5, 6, 7, 8, 15 und 16 wir beispielsweise derart eingestellt, dass der erste Dialysierflüssigkeitsfilter 3 seine Filterbetriebsart einnimmt. Hierzu wird das Magnetventil 5 geöffnet und die Magnetventile 4, 6, 7, 8, 15 und 16 werden geschlossen. Dann wird mittels der Dialysierflüssigkeitspumpe 1 Dialysierflüssigkeit 2 durch den ersten Dialysierflüssigkeitsfilter 3 in seiner Filterbetriebsart gefördert, eine zweite Stromaufnahme der elektrischen Dialysierflüssigkeitspumpe 1 gemessen und ein Zustand, beispielsweise in Form eines Verschleißes bzw. einer Verstopfung, der Filtermembran 9 des ersten Dialysierflüssigkeitsfilters 3 in Abhängigkeit von der gemessenen zweiten Stromaufnahme ermittelt.

Die gemessene zweite Stromaufnahme kann mit einem zweiten Schwellenwert verglichen werden. Falls die gemessene zweite Stromaufnahme größer als der zweite Schwellenwert ist, wird ein Fördervolumenstrom der Dialysierflüssigkeit 2 auf einen Maximalwert begrenzt, beispielswiese auf 80 % eines nominellen Fördervolumenstroms, und eine Empfehlung zum Wechseln der Filtermembran 9 des ersten Dialysierflüssigkeitsfilters 3 ausgegeben.

Der erste Schwellenwert und der zweite Schwellenwert können bei bekannten Referenzbedingungen ermittelt werden. Die erwartete zweite Stromaufnahme basiert auf der Erkenntnis, dass der Motorstrom durch den Membranwiderstand ansteigen sollte. Der gemessene Wert gibt Aufschluss auf den Zustand bzw. die Verschmutzung der Membran und damit auf die maximale Performance. Bei Überschreitung des zweiten Schwellenwerts wird die Therapie auf einen geringeren Dialysat-Fluss begrenzt und dem Anwender der Hinweis gegeben, den Dialysierflüssigkeitsfilter 3 zu wechseln.

Weiter kann die Magnetventilanordnung 4, 5, 6, 7, 8, 15 und 16 derart eingestellt werden, dass der erste Dialysierflüssigkeitsfilter 3 und der zweite Dialysierflüssigkeitsfilter 10 jeweils ihre Filterbetriebsart einnehmen und die Dialysierflüssigkeit durch den ersten und durch den zweiten Dialysierflüssigkeitsfilter 3 bzw. 10 gefördert wird. Hierzu wird das Magnetventil 6 geöffnet und die Magnetventile 4, 5, 7, 8, 15 und 16 werden geschlossen. Es wird dann eine dritte Stromaufnahme der elektrischen Dialysierflüssigkeitspumpe 1 gemessen und überprüft, ob die gemessene dritte Stromaufnahme in etwa das Zweifache der gemessenen zweiten Stromaufnahme beträgt. Falls die gemessene dritte Stromaufnahme nicht in etwa das Zweifache der gemessenen zweiten Stromaufnahme beträgt, wird ein Defekt des zweiten Dialysierflüssigkeitsfilters 10 festgestellt, vorausgesetzt, dass zuvor das erste Dialysierflüssigkeitsfilter 3 als funktionsfähig bestimmt worden ist. Andernfalls wird der zweite Dialysierflüssigkeitsfilter 10 als funktionsfähig bewertet.

Weiter kann die Magnetventilanordnung 4, 5, 6, 7, 8, 15 und 16 derart eingestellt werden, dass der erste Dialysierflüssigkeitsfilter 3 und der zweite Dialysierflüssigkeitsfilter 10 jeweils ihre Filterbetriebsart einnehmen und die Dialysierflüssigkeit durch den ersten Dialysierflüssigkeitsfilter 3, durch den zweiten Dialysierflüssigkeitsfilter 10 und intern durch den Substitutions-Port 12 gefördert wird. Hierzu werden die Magnetventile 7 und 8 geöffnet und die Magnetventile 4, 5, 6, 15 und 16 werden geschlossen. Es wird dann eine vierte Stromaufnahme der elektrischen Dialysierflüssigkeitspumpe 1 gemessen und überprüft, ob die gemessene vierte Stromaufnahme mit der der gemessenen dritten Stromaufnahme übereinstimmt. Falls die gemessene vierte Stromaufnahme nicht mit der der gemessenen dritten Stromaufnahme übereinstimmt, wir ein (Teil-) Verschluss des Substitutions-Ports 12 festgestellt. Die Vorrichtung 100 ist dann nicht einsatzbereit und muss technisch geprüft werden. Andernfalls wird der Substitutions-Port 12 als funktionsfähig bewertet.

Die Stromaufnahmen können bei einer Selbsttestprüfung aufgenommen und fortlaufend miteinander verglichen werden. Ein Anstieg über die Betriebszeit zeigt einen Verschleiß an und gibt damit einen Hinweis zum nächsten Wechselzeitpunkt der Dialysierflüssigkeitsfilter 3 und 10.

Am Ende einer Desinfektion kann ebenfalls die Stromaufnahme ermittelt werden. Das Ergebnis daraus gibt einen Hinweis, ob die Dialysierflüssigkeitsfilter 3 und 10 vollständig entkalkt worden sind bzw. die Desinfektion wiederholt werden müsste und/oder die Dialysierflüssigkeitsfilter 3 und 10 gewechselt werden sollten.

## Patentansprüche

1. Verfahren zur Zustandsüberwachung einer Vorrichtung (100) zur extrakorporalen Blutbehandlung, wobei die Vorrichtung (100) zur extrakorporalen Blutbehandlung aufweist:
- eine elektrische Dialysierflüssigkeitspumpe (1) zum Fördern von Dialysierflüssigkeit (2),
- einen ersten Dialysierflüssigkeitsfilter (3) mit einer Filtermembran (9), der in einer Spülbetriebsart und in einer Filterbetriebsart betreibbar ist, und
- eine Magnetventilanordnung (4, 5, 6, 7, 8) zur Steuerung eines Flusspfads der geförderten Dialysierflüssigkeit (2),
wobei das Verfahren die Schritte aufweist:
- Einstellen der Magnetventilanordnung (4, 5, 6, 7, 8) derart, dass der erste Dialysierflüssigkeitsfilter (3) seine Spülbetriebsart einnimmt,
- Fördern von Dialysierflüssigkeit (2) durch den ersten Dialysierflüssigkeitsfilter (3) in seiner Spülbetriebsart,
- Messen einer ersten Stromaufnahme der elektrischen Dialysierflüssigkeitspumpe (1),
- Vergleichen den gemessenen ersten Stromaufnahme mit einem ersten Schwellenwert und
- falls die gemessene erste Stromaufnahme größer als der erste Schwellenwert ist, Feststellen eines Defekts der elektrischen Dialysierflüssigkeitspumpe (1).

2. Verfahren nach Anspruch 1, **gekennzeichnet durch** die weiteren Schritte:
- Einstellen der Magnetventilanordnung (4, 5, 6, 7, 8) derart, dass der erste Dialysierflüssigkeitsfilter (3) seine Filterbetriebsart einnimmt,
- Fördern von Dialysierflüssigkeit (2) durch den ersten Dialysierflüssigkeitsfilter (3) in seiner Filterbetriebsart,
- Messen einer zweiten Stromaufnahme der elektrischen Dialysierflüssigkeitspumpe (1), und
- Ermitteln eines Zustands der Filtermembran (9) des ersten Dialysierflüssigkeitsfilters (3) in Abhängigkeit von der gemessenen zweiten Stromaufnahme.

3. Verfahren nach Anspruch 2, **gekennzeichnet durch** die weiteren Schritte:
- Vergleichen der gemessenen zweiten Stromaufnahme mit einem zweiten Schwellenwert und
- falls die gemessene zweite Stromaufnahme größer als der zweite Schwellenwert ist, Begrenzen eines Fördervolumenstroms der Dialysierflüssigkeit (2) auf einen Maximalwert und Ausgeben einer Empfehlung zum Wechseln der Filtermembran (9) des ersten Dialysierflüssigkeitsfilters (3) oder Ausgeben einer Empfehlung zum Wechseln des ersten Dialysierflüssigkeitsfilters (3).

4. Verfahren nach Anspruch 2 oder 3, **dadurch gekennzeichnet, dass**
- die Vorrichtung (100) zur extrakorporalen Blutbehandlung weiter aufweist:
- einen zweiten Dialysierflüssigkeitsfilter (10) mit einer Filtermembran (11), der in einer Spülbetriebsart und in einer Filterbetriebsart betreibbar ist,
wobei das Verfahren die Schritte aufweist:
- Einstellen der Magnetventilanordnung (4, 5, 6, 7, 8) derart, dass der erste Dialysierflüssigkeitsfilter (3) und der zweite Dialysierflüssigkeitsfilter (10) jeweils ihre Filterbetriebsart einnehmen und die Dialysierflüssigkeit durch den ersten und durch den zweiten Dialysierflüssigkeitsfilter gefördert wird,
- Messen einer dritten Stromaufnahme der elektrischen Dialysierflüssigkeitspumpe (1),
- Überprüfen, ob die gemessene dritte Stromaufnahme in etwa das Zweifache der gemessenen zweiten Stromaufnahme beträgt, und
- falls die gemessene dritte Stromaufnahme nicht in etwa das Zweifache der gemessenen zweiten Stromaufnahme beträgt, Feststellen eines Defekts des zweiten Dialysierflüssigkeitsfilters (10).

5. Verfahren nach Anspruch 4, **dadurch gekennzeichnet, dass**
- die Vorrichtung (100) zur extrakorporalen Blutbehandlung weiter aufweist:
- einen Substitutions-Port (12),
wobei das Verfahren die Schritte aufweist:
- Einstellen der Magnetventilanordnung (4, 5, 6, 7, 8) derart, dass der erste Dialysierflüssigkeitsfilter (3) und der zweite Dialysierflüssigkeitsfilter (10) jeweils ihre Filterbetriebsart einnehmen und die Dialysierflüssigkeit durch den ersten Dialysierflüssigkeitsfilter (3), durch den zweiten Dialysierflüssigkeitsfilter (10) und intern durch den Substitutions-Port (12) gefördert wird,
- Messen einer vierten Stromaufnahme der elektrischen Dialysierflüssigkeitspumpe (1),
- Überprüfen, ob die gemessene vierte Stromaufnahme mit der der gemessenen dritten Stromaufnahme übereinstimmt, und
- falls die gemessene vierte Stromaufnahme nicht mit der der gemessenen dritten Stromaufnahme übereinstimmt, Feststellen eines Teil-Verschlusses des Substitutions-Ports (12).

6. Verfahren nach einem der Ansprüche 3 bis 5, **dadurch gekennzeichnet, dass**
- der erste Schwellenwert und der zweite Schwellenwert bei bekannten Referenzbedingungen ermittelt werden.

7. Vorrichtung (100) zur extrakorporalen Blutbehandlung, die zur Durchführung des Verfahrens nach einem der vorhergehenden Ansprüche ausgebildet ist, aufweisend:
- eine elektrische Dialysierflüssigkeitspumpe (1) zum Fördern von Dialysierflüssigkeit (2),
- einen ersten Dialysierflüssigkeitsfilter (3) mit einer Filtermembran (9), der in einer Spülbetriebsart und in einer Filterbetriebsart betreibbar ist,
- eine Magnetventilanordnung (4, 5, 6, 7, 8) zur Steuerung eines Flusspfads der geförderten Dialysierflüssigkeit (2), und
- eine Steuereinheit (13), die den Betrieb der Vorrichtung (100) zur extrakorporalen Blutbehandlung derart steuert, dass ein Verfahren nach einem der vorhergehenden Ansprüche ausgeführt wird.
